# EUROPEAN PATENT SPECIFICATION

(11) **EP 1 906 875 B1**
(45) Date of publication and mention of the grant of the patent: **25.10.2017**
(21) Application number: 06711258.1
(22) Date of filing: 01.03.2006
(51) Int. Cl.: A61K 9/22, A61F 5/00

(54) **BIODEGRADABLE SELF-INFLATING INTRAGASTRIC IMPLANTS**
BIOLOGISCH ABBAUBARE SELBSTAUFBLASENDE MAGENIMPLANTATE
IMPLANTS GASTRIQUE BIODEGRADABLES A DEPLOIEMENT AUTOMATIQUE

(30) Priority: 01.03.2005 IL 16719405
(43) Date of publication of application: 09.04.2008
(73) Proprietor: Tulip Medical Ltd., 69620 Tel-Aviv (IL)
(72) Inventor: MARCO, Doron, 69620 Tel Aviv (IL)
(74) Representative: J A Kemp
(86) International application number: PCT/IL2006/000276
(87) International publication number: WO 2006/092789

(56) References cited:
- WO-A1-02/091961
- US-A- 3 674 014
- US-A- 4 607 618
- US-A1- 2003 109 935

## Description

The present invention generally relates to biodegradable self-inflating or self opening intragastric implants.

Obesity is a major health problem in developed countries. In the United States, the complications of obesity affect nearly one in five individuals at an annual cost of approximately $40 billion. Except for rare pathological conditions, weight gain is directly correlated to overeating.

Intragastric volume-occupying devices provide the patient a feeling of satiety after having eaten only small amounts of food. Thus, the caloric intake is diminished while the subject is satisfied with a feeling of fullness. Currently available volume- occupying devices have many shortcomings. For example, complex gastric procedures are required to insert some devices.

There has been ongoing clinical use of intragastric balloons for several years, and its success in the treatment of certain individuals with morbid obesity is well accepted.

United States Patent Application No. 2004/0186502 to Sampson et al. discloses a self- inflating intragastric volume-occupying balloon including a valve providing fluid communication into the balloon from outside. The '502 application further discloses a method for occupying stomach volume comprising the step of inserting the self- inflating intragastric balloon into the stomach, through the esophagus, while the balloon is deflated, then inflating the balloon by activating liquid influx through the aforesaid self-sealing valve.

United States Patent 4,607,618 to Angelchik discloses semi-rigid skeleton members, collapsible to a shape and dimensions suitable to be endoscopically inserted into the stomach through the esophagus and cardiac opening by means of feeding tube.

United States Patent 5,129,915 to Cantenys discloses a balloon intended to be swallowed and to occupy a given volume of the stomach in the presence of other balloons of the same type. This invention is designed to facilitate weight reduction of an individual and consists of an envelope containing two chemicals which can react with each other and with water at an elevated temperature in order to form a gas effecting the inflation of the balloon.

US 2003/0109935 A1 discloses an intragastric prosthesis for the treatment of morbid obesity.

There remains a long felt need for a biodegradable intragastric implant that can be delivered to the stomach by conventional oral administration and that controllably inflates after an approximate predetermined delay time.

It is thus an aim of the present invention to disclose a novel treatment for curbing appetite and/or treating obesity. This treatment is adapted for providing selective medical care and obesity therapy specifically suited to the ever-changing needs of an individual patient: wherein his eating habits; his daytime and nighttime behavior; his physiological and mental characteristics; his size and age are considered and effectively targeted.

Another aim of the present invention is to present a cost effective biodegradable self-inflating intragastric implant for curbing appetite and treating obesity, constructed from one or more discrete expandable and/or deformable members. According to the present invention, there is provided a biodegradable self-inflating or self opening intragastric implant, as defined in the independent claim.

### BRIEF DESCRIPTION OF THE FIGURES

In order to understand the invention and to see how it may be implemented in practice, a plurality of preferred embodiments will now be described, by way of non- limiting example only, with reference to the accompanying drawing, in which figures 1 to 16 present schematic illustrations of the implant. Figures 17 and 18 presenting a lateral cross-section and a top views, respectfully, of a lock mechanism comprising a connector locking a segment to a joint.

The following description is provided, alongside all chapters of the present invention, so as to enable any person skilled in the art to make use of the said invention and sets forth the best modes considered by the inventor for carrying out this invention. Various modifications, however, will remain apparent to those skilled in the art, since the generic principles of the present invention have been defined specifically to provide a biodegradable self-inflating intragastric implant for curbing appetite, constructed from one or more discrete expandable members and a treatment of curbing appetite by the same.

The appetite is curbed by a volume-occupying implant temporarily installed in the stomach of the patent. The installation is provided according to one embodiment of the present invention for a respectively short period of time (e.g., few hours) and/or heated to about 37<0>C.

This self-inflated implant is deployed in the stomach automatically, e.g., by utilizing shape memory materials, and/or orientation memory materials, shape memory alloys and/or orientation memory alloys or polymers or flexible materials adapted to expand themselves; utilizing biocompatible compositions; providing gas formation inflating the same or any combination thereof. It is acknowledged in this respect that at least a portion of the member is of a shape memory and/or orientation memory nature.

The term 'discrete expandable members' is related hereinafter to structural elements temporary interconnected only by a means of a biodegradable or disassembled tie, such that small debris is produced by the member which is easily evacuated from the stomach after use. Said members may be composed of biodegradable compositions, such as PLA, PGA, polysaccharides pastas, gelatins, nylons, especially nylon 12 etc.

The term "Pasta" refers hereinafter to mixtures of carbohydrates, e.g., flour and fluids, e.g., water, possibly with a predetermined measure of proteins, especially egg- related proteins.

The term "Gelatin" refers hereinafter to a protein product derived through partial hydrolysis of the collagen extracted from skin, bones, cartilage, ligaments, etc.

It is according to one embodiment of the present invention wherein the expandable elements are adapted to expand in vivo when they come into contact with gastric juice for a predetermined period of time and heated to about 37°C.

Said period of time may vary widely. For some purposes and in a non-limiting manner, it ranges from about 0.5 hour to 8 hours, preferably about 3 hours from initial contact with the gastric juice and/or at an elevated body temperature. The device is alternatively adapted to expand/form *in vivo* in a time being less than T2 and more than T1, wherein T1 is less than T2; where T1 is about 2 to 7 minutes; and where T2 is about 20 to 40 minutes.

For some other purposes, further in a non-limiting manner, it ranges from about 8 hours to about 24 hours. For other purposes, still in a non-limiting manner, it ranges from about 24 hours to about 96 hours. It is nevertheless acknowledged that longer periods of time are possible, such as 1 day to 30 days or more. Said predetermined period of time is selected by the specialist and reflects *inter alia* the size of the stomach (e.g. the age of the patient), food digestive parameters in the stomach and along the digestion tracks, conditions of the stomach and its sphincters, the content of the gastric juice etc, the patient's general physiological and mental condition etc. Said predetermined period of time may be equal intervals or may vary from time to time, from meal to meal etc.

The device is preferably provided wherein the self inflating device is further composed of at least one element selected from a group comprising a bio-absorbable element; bio-degradable element, disintegrating element or any combination thereof.

It is also according to one embodiment of the present invention wherein the device is self-inflated spontaneously and does not contain any inflating valves or the like, nor external inflating means.

It is also according to one embodiment of the present invention wherein at least a portion of the expandable elements are shape memory and/or orientation memory members being in their deflated state *ex vivo,* and further being in their native inflated state *in vivo* upon contact with gastric juice or when heated to the temperature inside the stomach for a predetermined period of time. The composition is selected in a non limiting manner form alloys, polymers, silicon or rubber-like materials etc.

It is also according to one embodiment of the present invention wherein at least a portion of the expandable elements are adapted to disassemble *in vivo* when in contact with gastric juice for a predetermined period of time.

It is also according to one embodiment of the present invention wherein the discrete expandable elements as defined in any of the above provide *in vivo* a two-dimensional sheet. The term sheet is related hereinafter to any 2D object. Said sheet may be provided in any size, shape or thickness. Hence, for example, oval thin sheets are useful, as well as polygonal double-layered ones. Said sheet may be smooth and/or comprise protruding patterns. The sheet may be provided tightly rolled, collapsed or otherwise forcedly packed inside a digestible capsule.

It is also according to one embodiment of the present invention wherein the device defined above is characterized *inter alia* by both a planar body portion and a frame portion.

It is also according to one embodiment of the present invention wherein the planar body portion is deployed *in vivo* so that a sheet-like plane is provided. The planar body portion can be twisted *in vivo* so that a bent, curved and/or folded object can be provided. Alternatively or additionally, the planar body portion is twisted *in vivo* so as to provide a coiled helix. Hence, the sidewalls of the stomach are in contact with the said flexible implant. The degree of the flexibility of the implant is provided by either the materials used in its construction or the manner said implant is opened or inflated *in vivo.* Moreover, the exact *in vivo* intragastric location of the implant is determined by those two factors of materials and structure when inflated.

It is also according to one embodiment of the present invention wherein the above mentioned planar body portion is selected from a group comprising *inter alia* a continuous membrane portion (e.g., a semi-permeable sheet, preventing solids or liquid from penetrating the implanted membrane), a mesh-like net or screen (e.g., a woven or knitted mesh, silicon materials, netting comprising many interlocking structural elements etc.), at least one aperture or open bore extending within the said planar body portion or any combination thereof.

It is also according to one embodiment of the present invention wherein the frame portion is selected from a group comprising at least one expandable and/or deformable rib being in its deflated state *ex vivo* and in its inflated state *in vivo*; and further wherein at least a portion of said ribs are interconnected by means of a tie such that a multi- dimensional inflated frame is provided. The term 'rib' is related hereinafter to any maneuverable, inflatable or expandable structural element of the device. Said ribs may be thin or thick, smooth or roughened, simple or shaped members. The ribs are comprised of one or more structural elements, and may be interconnected with one or more other ribs. Some of the ribs may be elastic (e.g., construction spreading ribs), some may include springs or spring-like means, some may be rigid, some may be and/or orientation memory materials, e.g., made of Nitinol, shape-memory polymeric compositions or a combination thereof.

It is also according to one embodiment of the present invention wherein at least a portion of the rib disassembles *in vivo.* This biodegradable characteristic may be provided by bringing the rib into contact with gastric juice for a predetermined period of time and/or heated to about 37°C. The terms 'disassemble' and/or 'biodegradable' are related hereinafter to a measurable activity of breaking down (pertaining to matter), decomposing under natural conditions characteristic of the stomach, taking apart, dismantling and/or coming apart. Those terms are especially related to activities provided in the stomach, whereat a low pH and a warm environment is provided. Other conditions however, are possible for providing said disassembling and/or biodegrading activities, such as the presence of one or more components of the gastric juice, additive coexistence etc.

It is also according to one embodiment of the present invention wherein the implant comprises a plurality of ribs providing the device's infrastructure. At least one portion of said ribs is interconnected with at least a portion of other ribs by a means of a tie.

The term 'tie' is related hereinafter to any joint, connection, link etc adapted to couple two or more structural elements. Said tie may be driven from physical, chemical or biological means.

It is also according to one embodiment of the present invention wherein one or more ties are disassembled *in vivo,* possibly a biodegradable tie or ties are disassembled by effective contact with gastric juice for a predetermined period of time and/or heated to about 37°C.

It is also according to one embodiment of the present invention wherein the discrete expandable elements form *in vivo* a three-dimensional object.

It is also according to one embodiment of the present invention wherein said ribs are characterized by proximal and distal ends. Said proximal end comprising a sliding means; wherein at least a portion of the ribs are being connected to at least one shaft by the said sliding means, such that the inflation of the structure *in vivo* is provided by sliding the said sliders along the said shaft or shafts. The sliding means could be fork-like surrounding at least a portion of the shaft. Said means may alternatively comprise a rotating hinge and/or at least one protruding element slidable in a recess located in the shaft etc.

It is also according to one embodiment of the present invention wherein the distal ends of the ribs comprise anchoring means, such that said ribs are interconnected with at least one end of the shaft by means of the said anchoring means.

It is also according to one embodiment of the present invention wherein the implant is deflated *ex vivo* and inflated *in vivo.* It is characterized by an umbrella-like structure and comprises a so-called umbrella-like assembly. This assembly comprises at least one shank, a plurality of slidable ribs and a canopy attached thereto. One or more ribs are adapted to slide along the main longitudinal axis of the shank while being connected to a pole of said shank. This unique implant's expanding mechanism is thus translating longitudinal 2D movement to a 3D lateral expanding maneuver.

It is thus in the scope of the present invention wherein at least a portion of the planar body defined above is provided as the canopy of the umbrella-like device.

It is also according to one embodiment of the present invention wherein the implant is a self-inflated structure comprising two or more umbrella-like assemblies and/or two or more interconnected umbrellas. Thus for example, the multi-component structure comprising two umbrella-assemblies: a first assembly is adapted to inflate towards one pole of the shank, and said second assembly is adapted to inflate towards the opposite pole of the shank; the assemblies are either separated or interconnected. Hence, a conch-like close or open structure is obtained.

It is also according to one embodiment of the present invention wherein at least a part of the planar body portion is adapted to disassemble *in vivo.* Preferably, yet not exclusively, the planar body portion is adapted to disassemble *in vivo* when in contact with gastric juice for a predetermined period of time and/or heated to about 37°C.

It is also according to one embodiment of the present invention, wherein the discrete expandable elements provided *in vivo* a three-dimensional bladder. The bladder comprises a body portion and infrastructural elements which are deflated *ex vivo* and inflated *in vivo.* The 3D shape of the bladder when inflated *in vivo* is selected from a group comprising, in a non-limiting manner, balloon, football-like, basketball-like polygonal bag-shaped construction, hose-like, stent-like or ring-like shape, floating pot-like objects, floating on the upper level of the gastric juice, coiled helix or any combination thereof. The terms football and basketball are denoted to approximately round balloon-like implants, wherein the infrastructure or frame provided by the discrete structural elements is provided by obtaining hexagonal/pentagonal elements (so called 'football' structure) or concaved curvatures and arcs (e.g., basketball structure).

It is also according to one embodiment of the present invention, wherein the bladder is constructed by a means of interconnected structural elements. Said elements are at least partially disassembled *in vivo.* Preferably, the elements are at least partially disassembled *in vivo* when in contact with gastric juice for a predetermined period of time and/or heated to about 37°C.

It is also according to one embodiment of the present invention, wherein the bladder is constructed by means of structural elements interconnected by mutual joints. The joints are at least partially disassembled *in vivo.* Preferably, the joints are at least partially disassembled *in vivo* when in contact with gastric juice for a predetermined period of time and/or heated to about 37°C. Moreover, the bladder is potentially and at least partially disassembled *in vivo.* Preferably, when in contact with gastric juice for a predetermined period of time and heated to about 37°C.

It is also according to one embodiment of the present invention, wherein a swallowable device as defined in any of the above is disclosed. This swallowable is adapted (a) to be administrated orally to the patient in its collapsed configuration, (b) to be transported to the stomach in its deflated state, (c) to expand therein up to a predetermined size, (d) after a predetermined period of time and/or heated to about 37°C to disassemble and (e) to be then totally evacuated from the stomach.

It is still according to another embodiment of the present invention, wherein an implantable device according to any of the above is disclosed. This implantable device is adapted (a) to be implanted via the mouth of the patient in its collapsed configuration, (b) to be transported to the stomach in its deflated state, (c) to expand therein up to a predetermined size, (d) after a predetermined period of time and/or heated to about 37°C to disassemble; and (e) to be totally evacuated from the stomach.

It is also according to one embodiment of the present invention, wherein one or more of the above defined discrete expandable members are made of materials selected from nickel titanium alloys (nitinol); polymeric compositions, such as polyglycolide, poly 1-lactic (PLA) poly glycolide acid (PGA) or any PLA-PGA mixture thereof; poly(.xi.-caprolactone), poly(dioxanone), poly(glycolide-co-trimethylene carbonate), poly(hydroxybutyrate-co-hydroxyvalerate), polyglyconate polyanhydrides or polyorthesters; polydioxanone, organic materials, such as protein based compositions; inorganic materials such as double or triple salt compositions, radiopaque materials or any mixture thereof. Commerically available materials are also useful for providing degradable implants, e.g., Vicryl® by Ethicon. The expandable members are preferably, yet not exclusively coated with at least one layer comprising slow-released therapeutic compositions, biocompatible coating materials, biological glues etc.

It is also according to one embodiment of the present invention, wherein the body of the self-inflated device is made of materials selected in a non-limiting manner from polymeric compositions, such as the PLA-PGA compositions and others defined above, organic or inorganic materials (such as anhydrated carbohydrates known in the art, commercially available polyethylene glycol, pure dried silica gels etc) gels, hydrogels, hydratable fillers, swelling agents, organic compositions, inorganic compositions, dry powders or any mixture thereof.

It is also according to one embodiment of the present invention, wherein the body of the device comprises compositions selected from a group comprising *inter alia* slow-release therapeutic compositions, medication, pH buffers, anti-acid compositions, anti-inflammatory agents, anti histamines, additives, lubricants, UV contrasting agents, test and small agents, digestive-related therapeutic agents, probiotic bacteria cultures or any combination thereof.

It is also according to one embodiment of the present invention, wherein the implant as defined in any of the above is coated by a digestible capsule or the like, adapted to facilitate an easy introduction of the device into the stomach.

Another mechanism for inflating the implant is provided by gas formation due to chemical or biological reactions. Thus, for example, carbon dioxide formation is provided by admixing an effective measure of sodium bicarbonate and potassium bicarbonate with the gastric juice, comprising *inter alia* water soluble acid at a very low pH. A method of curbing the appetite is disclosed. This method comprises introducing a biodegradable self-inflating intragastric implant, constructed from one or more discrete expandable members. It is preferably according to another embodiment, wherein the method is provided by using the implant as defined in any of the above.

The method preferably comprises the following steps: (a) applying a biodegradable self-inflating intragastric implant in its *ex vivo* deflated state; (b) introducing the deflated implant into the stomach of a patient; e.g., via the esophagus (c) inflating the same *in vivo* to a predetermined size and shape; (d) after a predetermined period of time and/or heated to about 37°C, disassembling the same; (e) evacuating the disassembled debris outside the stomach of the patient.

The method comprising swallowing the intragastric implant in its *ex vivo* deflated state. Alternatively, the method comprising endoscopically implanting the intragastric implant in its *ex vivo* deflated state into the stomach of the patient. A novel treatment for curbing appetite is provided. This treatment comprises a periodic oral administration of biodegradable self-inflating intragastric implant constructed from one or more discrete expandable members. The intervals between each administration are determined by an expert and are in correlation with both physiological and mental characteristics of the patient. This treatment is especially useful wherein the deflated implant is swallowed before meals such that the food intake during the meal and afterwards is decreased.

It is lastly according to another embodiment, wherein the aforesaid treatment is comprised of administration of swallowable and biodegradable self-inflating implants as defined in any of the above, wherein the implants used in the treatment vary in size and/or shape.

Hence, for example, treatment is initiated by administrating implants that are inflated *in vivo* to small intragastric objects, e.g., balloons of small external diameter, folded 2D planar insertions or sheet-like implants, floating balloons or a combination thereof. In the course of the treatment, said implants are administrated such that their final *in vivo* volume and size is respectively bigger and/or its shaped so that their rim is leaning or pushing to a predetermined measure the sidewalls of the stomach, e.g., by utilizing bigger balloons, bladders, 3D helix-like insertions etc. After a physiological steady state is obtained, other implants according to the present invention are usable, such as floating pot-like insertions, meshed-wall devices etc. At the end of said acute treatment, maintaining treatment may employ other especially designed implants.

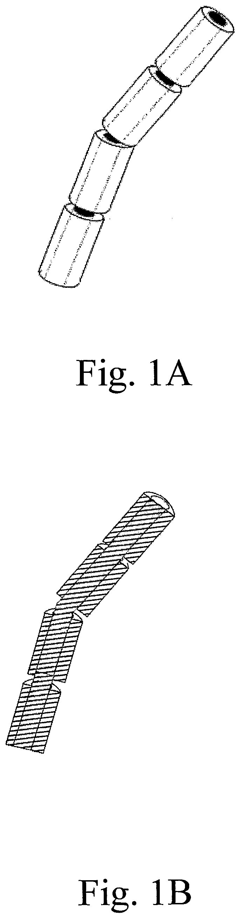

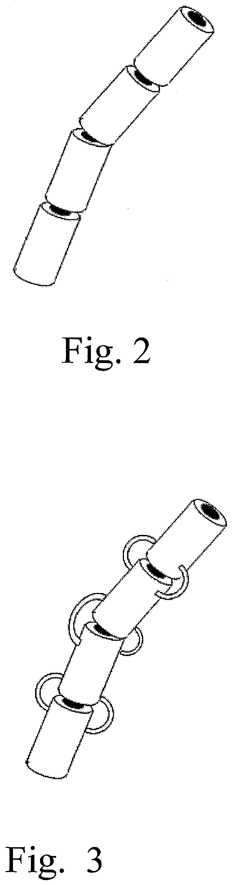

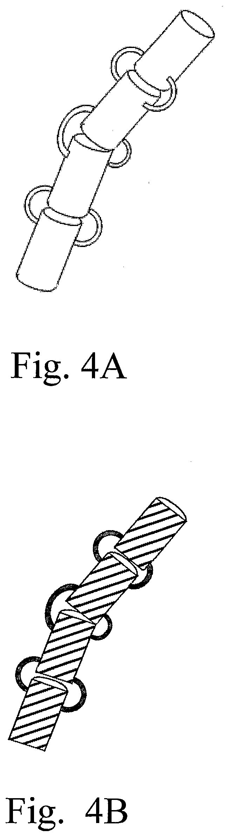

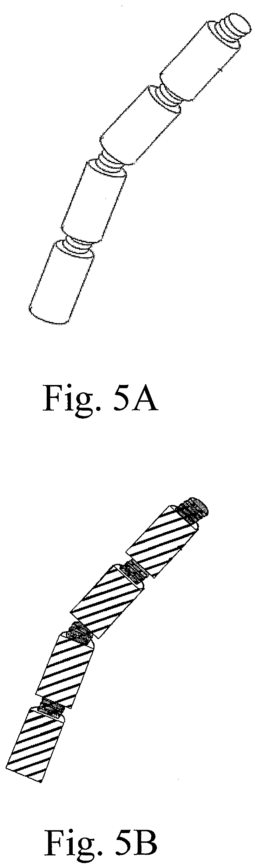

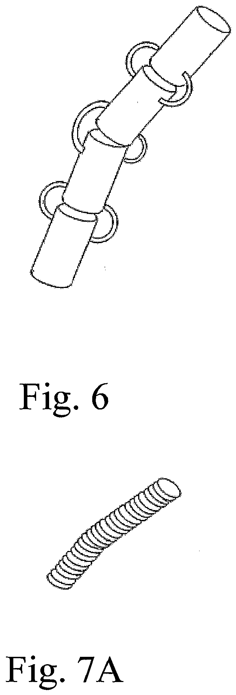

Reference is made now to the figures, illustrating a few possible embodiments of the present invention, wherein figure 1A presents a flexible biodegradable thread comprising shape memory and/or orientation memory characteristics and one or more ingredients located in its inner volume, figure 1B presents a cross section of the same. In figures 1A and 1B the tubes are made of biodegradable material (like pasta tubes), the connection between them is of a biodegradable rubber-like material, such as chewing gum or similar materials. Figure 2 illustrates similar tubes wherein the tube and it's a cylinder materials and the connection between them is rubber like material. Figure 3 illustrates similar tubes additionally comprising a number of strengthening biodegradable wires attached to the sides of the tube. Figure 4A illustrates similar embodiment wherein the strengthening biodegradable wires are extended from the sides, figure 4B illustrates a cross section of the same. Figure 5A illustrates similar embodiment wherein instead of the rubber defined above, a spring-like member is extended throughout the biodegradable materials, figure 5B illustrates a cross section of the same. Figure 6 illustrates similar embodiment as illustrated in Figure 4A. It is acknowledged in this respect that biodegradable members are selected from the tube-like portions, the reinforcement members or any combination thereof. All members are preferably yet not exclusively constructed in a manner that flexible and non-breaking apart fragments and members are provided.

It is acknowledged in this respect that biodegradable members may be coupled to the tube-like portions, the reinforcement members or any combination thereof. All members are preferably yet not exclusively constructed in a manner that flexible and non-breaking apart fragments and members are provided.

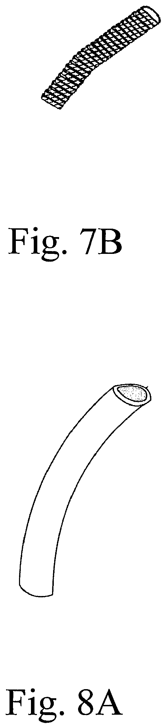

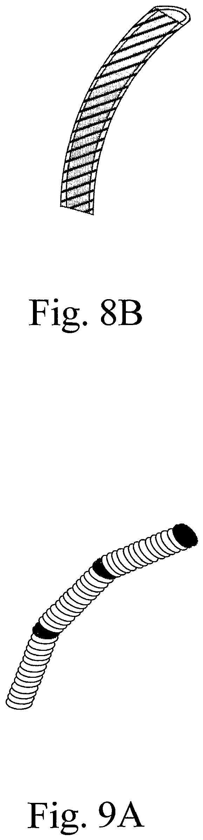

Figure 7A illustrates similar embodiment wherein the implant is one spring-like member made of biodegradable materials. Figure 7B illustrates a cross-section of the same. Figure 8A illustrates a hollow flexible biodegradable tube full of gas. The tube is filled with a gas. When the tube is accommodated in a warm body it expands to its predetermine shape. Figure 8B illustrates a cross section of the same. Figure 9A illustrates an embodiment wherein the spring and the rubber materials are described.

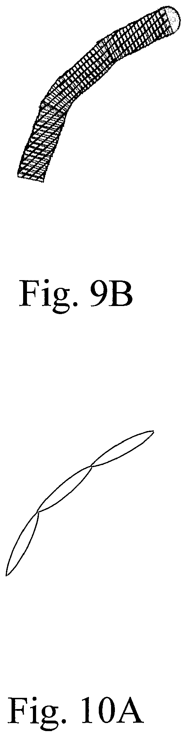

Figures 9 disclose a multiple spring-like members interconnected in its longitudinal axis. Figure 9B illustrates a cross section of the same.

It is in the scope of the present invention wherein the swallowable implant as defined in the appended claims further comprises at least one segment having an inner volume, said volume comprises a compressed gas adapted to inflate said device *in vivo.* The gas may be generated *in vivo,* e.g., by diluting gas formatting materials (calcium carbonate for example) in the intragastric solution or otherwise. Alternatively or additionally, said gas may be compressed or generated ex vivo before swallowing or implanting the said device. The gas formation may also be provided by reacting two or more compositions in vivo so as to expose at least one gas generation composition to the gastric fluid, such as providing a number of apertures in a membrane (of biodegradable nature for example) enveloping said gas-forming composition.

The present description also discloses a method of curbing appetite that comprises the steps of applying a biodegradable self-inflating intragastric implant in its *ex vivo* deflated state; introducing the deflated implant into the stomach of a patient; inflating the same *in vivo* to a predetermined size and shape by means of pressing a gas inside an inner portion of the implant such that said gas provided said device's *in vivo* inflating; after a predetermined period of time and/or heated to about 37°C, disassembling the same and evacuating the disassembled debris outside the stomach of the patient.

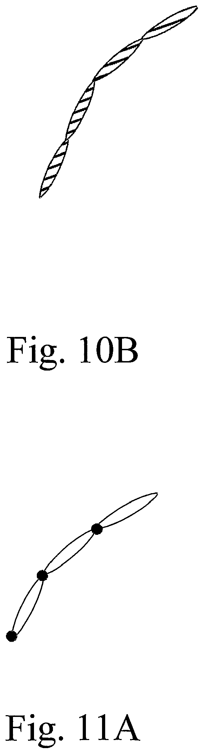

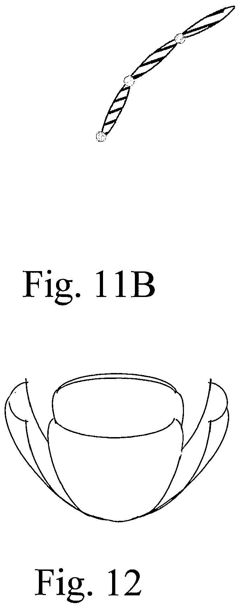

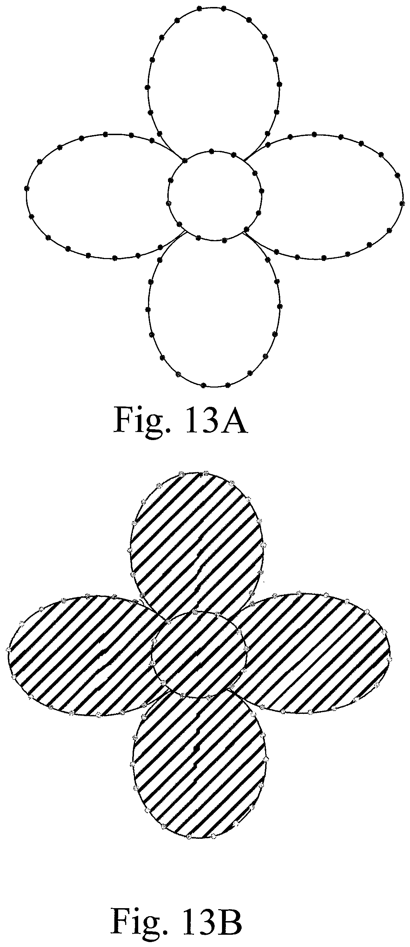

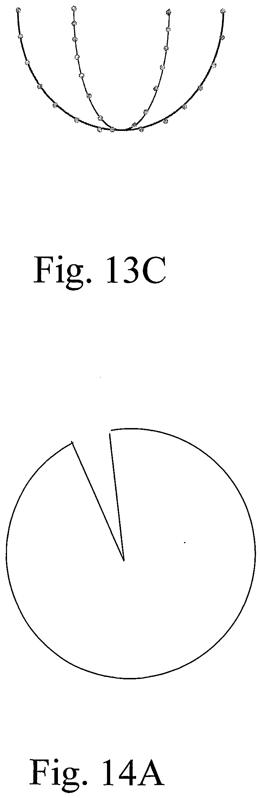

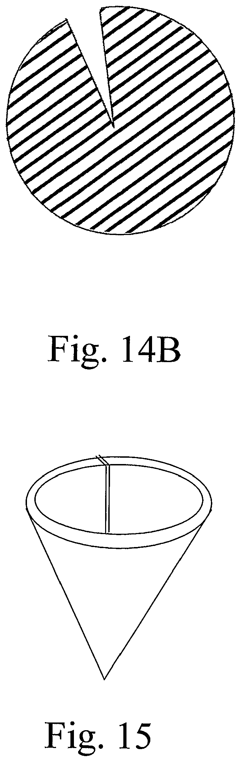

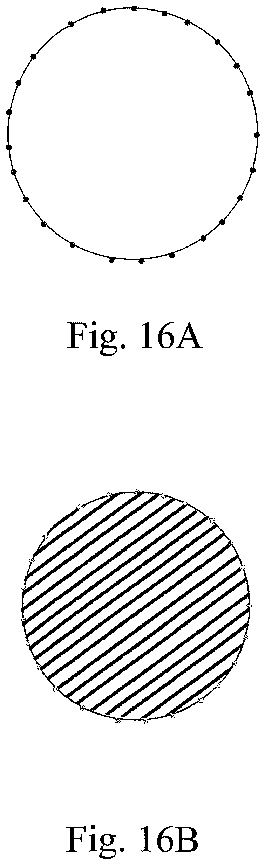

Figure 10A discloses a snare-like or other shape that is adapted to jump back to its original shape after folding. Figure 10B discloses a cross-section of the same. Figure 11A discloses the same in its expanded configuration. Figure 11B discloses a cross section of the same. Figure 12 presents a cup-like implant in its folded unexpanded configuration. Figure 13A presents the same in the deployed configuration, e.g., wherein the sheet-like member is made of nylon-like materials, and especially pH sensitive materials adapted to pill after being immersed for a predetermined time in the gastric environment. Figure 13B illustrates vertical cross section of the same. Figure 13C illustrates horizontal cross section of the same. Figure 14A presents a circular implant in its collapsed conformation. Figure 14B illustrates a cross section of the same. Figure 15 presents the same in its rolled structure, adapted to gain only a narrow diameter such that its intragastric application is enabled. The circumference of said implant may comprise of a number of detaching members, as illustrates in Fig. 16A, providing an *in vivo* disassembling of the implant after a predetermined time. Figure 16B represents a cross section of the same. Said members are made, for example, of PLA coated with edible compositions, such as polysaccharides, pasta, gelatin, nylon 12, nylon 6 or any mixture thereof.

It is in the scope of the present invention wherein at least a portion of the segments are interconnected by means of at least one connector, being either disposable (degradable) or not. Said connectors are of various shapes and sizes, and selected from hooks, mortar and mortar structures, wire or chain-like connections or any other joint known in the art.

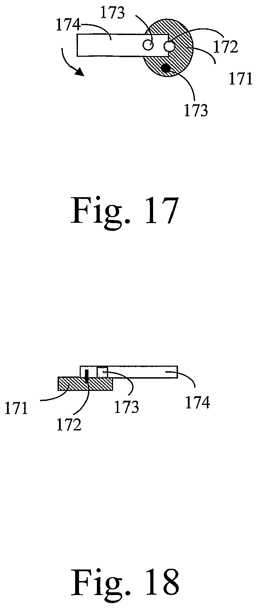

Reference is made now to figures 17 and 18 presenting a lateral cross-section and a top views, respectfully, of a contactor according one embodiment of the present invention. Said contactor comprising inter alia a joint 171, here a plastic-made (e.g., PLA-PGA biocompatible and biospendable material) circular member having an axis (172) in which segment 174 is rotating, said segment comprising at least one aperture (173), which fits in size and shape perturbing member (173), such that by rotating the segment to a certain measure (here, about 90 degrees), segment 174 is lock on joint 171. Said segment is preferably yet not exclusively composed of PLA-PGA materials, wherein joint 171 is coated by a silicone containing layer. Said locking mechanism is adapted to reversibly immobilize each of a portion of the segments to a predetermined 3D orientation when placed into the gastric juice, before disassembling.

It is further in the scope of the present invention wherein the self-inflating intragastric implant is assembled and/or disassembled (biodegraded) in a series of following or simultaneous events. Said implant thus may comprise more than one type of biodegradable ingredients. For example, the ingredients may be constructed of segments or layers or coatings of various biodegradability measure such that an *in vivo* non-linear disassembling is provided.

## Claims

1. Biodegradable self-inflating or self opening intragastric implant for curbing appetite, constructed from one or more discrete expandable, stretchable and/or deformable members; having a collapsed configuration and an expanded configuration; wherein said implant is swallowable, adapted to be administrated orally by a patient in said collapsed configuration, to be transported to the stomach, to expand therein up to a predetermined size and after a predetermined period of time to disassemble and then to be totally evacuated from the stomach;
**characterized in that** the expandable members are adapted to expand in vivo when in contact with gastric juice for a predetermined period of time.

2. The implant according to claim 1, wherein the expandable elements are adapted to expand in vivo when heated to about 37 degrees centigrade.

3. The implant according to claim 1, wherein at least a portion of the expandable elements are shape memory members and/or orientation memory being in their deflated state ex vivo, and further being in their native inflated state in vivo when in contact with gastric juice for a predetermined period of time or heated to the in vivo body temperature.

4. The implant according to claim 1, wherein at least a portion of the expandable elements are adapted to disassemble in vivo when in contact with gastric juice for a predetermined period of time and/or exposed to the in vivo elevated temperature.

5. The implant according to claim 1, wherein the discrete expandable elements provided in vivo a two-dimensional sheet.

6. The implant according to claim 5 characterized inter alia by both a planar body portion and a frame portion.

7. The implant according to claim 6, wherein the planar body portion is deployed in vivo so as a sheet-like plane is provided.

8. The implant according to claim 6, wherein the planar body portion is twisted in vivo so that a bent, curved and/or folded object is provided.

9. The implant according to claim 6, wherein the planar body portion is twisted in vivo so that a coiled helix is provided.

10. The implant as defined in claim 6 or any of its dependent claims, wherein the planar body portion is selected from a group comprising a continuous membrane portion, a mesh-like net or screen, at least one aperture or open bore exceeding therein said planar body portion or any combination thereof.

11. The implant as defined in claim 6 or any of its dependent claims, wherein the frame portion is selected from a group comprising at least one expandable and/or deformable rib being in its deflated state ex vivo and in its inflated state in vivo; and further wherein at least a portion of said ribs are interconnected by means of a tie and/or a shape memory and/or orientation memory connector such that a multi-dimensional inflated frame is provided.

12. The implant according to claim 11, wherein at least one portion of the rib disassembles in vivo.

13. The implant according to claim 12, wherein at least one portion of the rib disassembles in vivo on being in contact with gastric juice for a predetermined period of time.

14. The implant according to claim 11, comprising a plurality of ribs providing the device's infrastructure; wherein at least one portion of said ribs is interconnected with at least one portion of other ribs by a means of a tie.

15. The implant according to claim 14, wherein one or more ties, shape memory and/or orientation memory connectors are disassembled in vivo.

16. The implant according to claim 14, wherein one or more ties are disassembled in vivo when said tie or ties are being contacted with gastric juice for a predetermined period of time and/or heated to about 37 degrees centigrade.

17. The implant according to claim 1, adapted to disassemble in a time lower than 72 hours from initially coming into contact with the gastric juice and/or heated to about 37 degrees centigrade.

18. The implant according to claim 17, adapted to disassemble in a time ranging from about 1 to 24 hours from initially coming into contact with the gastric juice and/or heated to about 37 degrees centigrade.

19. The implant according to claim 17, adapted to disassemble in a time ranging from about 3 hours from initially coming into contact with the gastric juice.

20. The implant according to claim 19, said the device is adapted to expand/form in vivo in a time being less than T2 and more than T1, wherein T1 is less than T2; where T1 is about 2 to 7 minutes; and where T2 is about 20 to 40 minutes.

## Patentansprüche

1. Biologisch abbaubares selbstaufblasendes oder selbstöffnendes Magenimplantat zum Zügeln des Appetits, hergestellt aus einem oder mehreren einzelnen ausdehnbaren, dehnbaren und/oder verformbaren Teilen; mit einer kollabierten Konfiguration und einer ausgedehnten Konfiguration; wobei das Implantat schluckbar und angepasst ist, um einem Patienten in der kollabierten Konfiguration oral verabreicht zu werden, zu dem Magen transportiert zu werden, darin auf eine festgelegte Größe ausgedehnt zu werden und nach einem festgelegten Zeitraum zerlegt und dann vollständig aus dem Magen entfernt zu werden;
**dadurch gekennzeichnet, dass** die ausdehnbaren Teile angepasst sind, um sich in vivo auszudehnen, wenn sie über einen festgelegten Zeitraum mit Magensäure in Kontakt kommen.

2. Implantat nach Anspruch 1, wobei die ausdehnbaren Elemente angepasst sind, um sich in vivo auszudehnen, wenn sie auf über 37 Grad Celsius erwärmt werden.

3. Implantat nach Anspruch 1, wobei mindestens ein Abschnitt der ausdehnbaren Elemente Formgedächtnisteile und/oder Ausrichtungsgedächtnisteile sind, die ex vivo in ihrem entleerten Zustand sind und ferner in vivo in ihrem ursprünglichen aufgeblasenen Zustand sind, wenn sie über einen festgelegten Zeitraum mit Magensaft in Kontakt kommen oder wenn sie auf die in vivo Körpertemperatur erwärmt sind.

4. Implantat nach Anspruch 1, wobei mindestens ein Abschnitt der ausdehnbaren Elemente angepasst ist, um sich in vivo zu zerlegen, wenn er über einen festgelegten Zeitraum mit Magensaft in Kontakt kommt und/oder wenn er der in vivo erhöhten Temperatur ausgesetzt ist.

5. Implantat nach Anspruch 1, wobei die in vivo bereitgestellten einzelnen ausdehnbaren Elemente eine zweidimensionale Platte sind.

6. Implantat nach Anspruch 5, unter anderem durch einen ebenen Körperabschnitt und einen Rahmenabschnitt gekennzeichnet.

7. Implantat nach Anspruch 6, wobei der ebene Körperabschnitt in vivo eingesetzt ist, damit eine plattenartige Ebene bereitgestellt ist.

8. Implantat nach Anspruch 6, wobei der ebene Körperabschnitt in vivo so gedreht ist, dass ein gebogenes, gekrümmtes und/oder gefaltetes Objekt bereitgestellt ist.

9. Implantat nach Anspruch 6, wobei der ebene Körperabschnitt in vivo so gedreht ist, dass eine spiralisierte Helix bereitgestellt ist.

10. Implantat wie in Anspruch 6 oder einem der davon abhängigen Ansprüche definiert, wobei der ebene Körperabschnitt aus einer Gruppe, umfassend einen zusammenhängenden Membranabschnitt, ein maschenartiges Netz oder ein Sieb, mindestens eine Öffnung oder einen offenen Durchlass, die/der darin über den ebenen Körperabschnitt hinausragt, oder eine Kombination davon, gewählt ist.

11. Implantat wie in Anspruch 6 oder einem der davon abhängigen Ansprüche definiert, wobei der Rahmenabschnitt aus einer Gruppe, umfassend mindestens eine ausdehnbare und/oder verformbare Rippe, die ex vivo in ihrem entleerten Zustand und in vivo in ihrem aufgeblasenen Zustand ist; und wobei ferner mindestens ein Abschnitt der Rippen durch eine Bindung und/oder einen Formgedächtnis- und/oder Ausrichtungsgedächtnisverbinder so verbunden ist, dass ein multidimensionaler aufgeblasener Rahmen bereitgestellt ist, gewählt ist.

12. Implantat nach Anspruch 11, wobei sich mindestens ein Abschnitt der Rippe in vivo zerlegt.

13. Implantat nach Anspruch 12, wobei sich mindestens ein Abschnitt der Rippe in vivo zerlegt, wenn er über einen festgelegten Zeitraum mit Magensaft in Kontakt kommt.

14. Implantat nach Anspruch 11, umfassend eine Vielzahl von Rippen, die die Infrastruktur der Vorrichtung bereitstellen; wobei mindestens ein Abschnitt der Rippen mit mindestens einem Abschnitt anderer Rippen durch eine Bindung verbunden ist.

15. Implantat nach Anspruch 14, wobei eine oder mehrere Bindungen, Formgedächtnis- und/oder Ausrichtungsgedächtnisverbinder in vivo zerlegt werden.

16. Implantat nach Anspruch 14, wobei eine oder mehrere Bindungen in vivo zerlegt werden, wenn die Bindung oder die Bindungen über einen festgelegten Zeitraum mit Magensaft in Kontakt gebracht werden und/oder wenn sie auf etwa 37 Grad Celsius erwärmt werden.

17. Implantat nach Anspruch 1, angepasst, um sich in einer Zeit von weniger als 72 Stunden ab dem ersten Kontakt mit dem Magensaft und/oder ab dem Erhitzen auf etwa 37 Grad Celsius zu zerlegen.

18. Implantat nach Anspruch 17, angepasst, um sich in einer Zeit von einer Stunde bis zu 24 Stunden ab dem ersten Kontakt mit dem Magensaft und/oder ab dem Erhitzen auf etwa 37 Grad Celsius zu zerlegen.

19. Implantat nach Anspruch 17, angepasst, um sich in einer Zeit ab etwa 3 Stunden nach dem ersten Kontakt mit dem Magensaft zu zerlegen.

20. Implantat nach Anspruch 19, wobei die Vorrichtung angepasst ist, um sich in vivo in einer Zeit von weniger als T2 und mehr als T1 auszudehnen/zu formen, wobei T1 weniger als T2 ist; wobei T1 etwa 2 bis 7 Minuten ist und wobei T2 etwa 20 bis 40 Minuten ist.

## Revendications

1. Implant intragastrique autogonflable et à ouverture automatique biodégradable destiné à limiter l'appétit, conçu à partir d'au moins un élément dépliable, étirable et/ou déformable discret ; présentant une configuration repliée et une configuration dépliée ; ledit implant pouvant être avalé, étant adapté pour être administré par voie orale par un patient dans ladite configuration repliée, être transporté jusqu'à l'estomac, s'y déplier jusqu'à une taille prédéterminée, puis, après une durée prédéterminée, se décomposer et être intégralement évacué de l'estomac ;
**caractérisé en ce que** les éléments dépliables sont adaptés pour se déplier en vivo au contact des sucs gastriques pendant une durée prédéterminée.

2. Implant selon la revendication 1, dans lequel les éléments dépliables sont adaptés pour se déplier in vivo lorsqu'ils sont chauffés à environ 37 degrés Celsius.

3. Implant selon la revendication 1, dans lequel au moins une partie des éléments dépliables sont des éléments à mémoire de forme et/ou à mémoire d'orientation dans leur état dégonflés ex vivo, et sont en outre dans leur état gonflé in vivo au contact des sucs gastriques pendant une durée prédéterminée ou chauffés à la température corporelle in vivo.

4. Implant selon la revendication 1, dans lequel au moins une partie des éléments dépliables sont adaptés pour se décomposer in vivo au contact des sucs gastriques pendant une durée prédéterminée et/ou exposés à la température élevée in vivo.

5. Implant selon la revendication 1, dans lequel les éléments dépliables discrets forment in vivo une feuille bidimensionnelle.

6. Implant selon la revendication 5, caractérisé, entre autres, par à la fois une partie de corps et une partie de cadre.

7. Implant selon la revendication 6, dans lequel la partie de corps plane est déployée in vivo de sorte qu'un plan de type feuille soit formé.

8. Implant selon la revendication 6, dans lequel la partie de corps plane est tordue in vivo de sorte à former un objet coudé, courbé et/ou plié.

9. Implant selon la revendication 6, dans lequel la partie de corps plane est tordue in vivo de sorte à former un enroulement en hélice.

10. Implant tel que défini dans la revendication 6 ou l'une quelconque de ses revendications dépendantes, dans lequel la partie de corps plane est choisie dans un groupe comprenant une partie de membrane continue, un filet ou tamis de type treillis, au moins une ouverture ou un alésage ouvert s'y trouvant et dépassant ladite partie de corps plane, ou une l'une quelconque de leurs combinaisons.

11. Implant tel que défini dans la revendication 6 ou l'une quelconque de ses revendications dépendantes, dans lequel la partie de cadre est choisie dans un groupe comprenant au moins une nervure dépliable et/ou déformable se trouvant dans son état dégonflé ex vivo et dans son état gonflé in vivo ; et en outre dans lequel au moins une partie desdites nervures étant raccordées entre elles au moyen d'un lien et/ou d'un raccord à mémoire de forme et/ou à mémoire d'orientation de sorte à former un cadre gonflé multidimensionnel.

12. Implant selon la revendication 11, dans lequel au moins une partie de la nervure se décompose in vivo.

13. Implant selon la revendication 12, dans lequel au moins une partie de la nervure se décompose in vivo au contact des sucs gastriques pendant une durée prédéterminée.

14. Implant selon la revendication 11, comprenant une pluralité de nervures formant l'infrastructure du dispositif ; au moins une partie desdites nervures étant raccordées avec au moins une partie d'autres nervures au moyen d'un lien.

15. Implant selon la revendication 14, dans lequel au moins un lien, raccord à mémoire de forme et/ou à mémoire d'orientation est décomposé in vivo.

16. Implant selon la revendication 14, dans lequel au moins un lien est décomposé in vivo lorsque ledit au moins un lien est mis en contact avec les sucs gastriques pendant une durée prédéterminée et/ou chauffé à environ 37 degrés Celsius.

17. Implant selon la revendication 1, adapté pour se décomposer dans un temps inférieur à 72 heures à partir de la mise en contact avec les sucs gastriques et/ou du chauffage à environ 37 degrés Celsius.

18. Implant selon la revendication 17, adapté pour se décomposer dans un temps allant d'environ 1 à environ 24 heures à partir de la mise en contact avec les sucs gastriques et/ou du chauffage à environ 37 degrés Celsius.

19. Implant selon la revendication 17, adapté pour se décomposer dans un temps à partir d'environ 3 heures à partir de la mise en contact avec les sucs gastriques.

20. Implant selon la revendication 19, ledit dispositif étant adapté pour se déplier/former in vivo dans un temps inférieur à T2 et supérieur à T1, T1 étant inférieur à T2 ; où T1 vaut environ 2 à 7 minutes ; et où T2 vaut environ 20 à 40 minutes.
